# EUROPEAN PATENT SPECIFICATION

(11) **EP 3 434 309 B1**
(45) Date of publication and mention of the grant of the patent: **29.06.2022**
(21) Application number: 18195848.9
(22) Date of filing: 07.07.2016
(51) Int. Cl.: A61M 25/00, A61M 25/10

(54) **CATHETER**
KATHETER
CATHÉTER

(30) Priority: 27.10.2015 JP 2015210511
(43) Date of publication of application: 30.01.2019
(62) Divisional of application: 16178436.8
(73) Proprietor: ASAHI INTECC CO., LTD., Seto-shi, Aichi 489-0071 (JP)
(72) Inventor: SHIMIZU, Hirotomo, Seto-shi, Aichi 489-0071 (JP); ISHIKAWA, Masatomo, Seto-shi, Aichi 489-0071 (JP)
(74) Representative: Winter, Brandl - Partnerschaft mbB

(56) References cited:
- EP-A1- 2 174 685
- EP-A2- 0 807 444
- WO-A1-2007/067324
- US-A1- 2010 004 593
- US-A1- 2013 018 318

## Description

### TECHNICAL FIELD

The present invention relates to a catheter to be inserted into a tubular organ in human body such as a blood vessel, a gastrointestinal tract and a ureter as well as into a body tissue.

### BACKGROUND ART

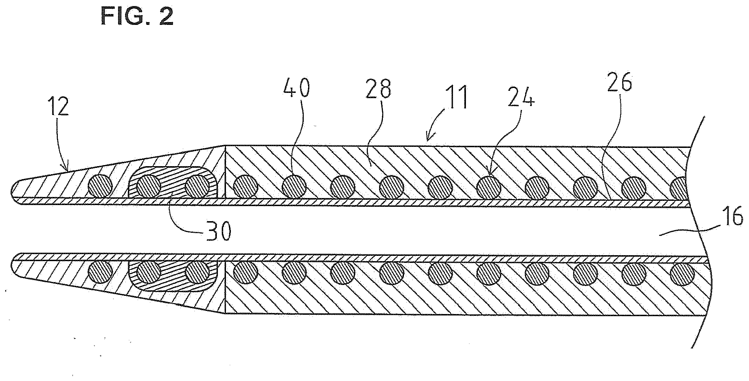

As a catheter to be inserted into a tubular organ in human body such as a blood vessel, a gastrointestinal tract and a ureter as well as into a body tissue, known is a catheter having a braid thereinside, the braid comprising two or more metal element wires. For example, JP 2014-188337 A describes a catheter 100 comprising a tubular body 10, a front end tip 58 attached to the front end of the tubular body 10 and a reinforcement wire 32 arranged inside the tubular body 10 and the front end tip 58 (see Fig. 2 and the like).

In this type of a catheter, the stiffness of the catheter itself is enhanced because a braid is arranged inside the tubular body 10 (hereinafter referred to as the "catheter body") and the front end tip 58, which can prevent the inner cavity of the catheter from collapsing. Further, in a case where the braid in place is replaced which a coil body, the torque transmissibility from the base end of the catheter to the front end will be increased.

US 2010/004593 A1 discloses a balloon catheter comprising a catheter body having a lumen, a resin front end tip having a lumen communicating with the lumen of the catheter body and attached to a front end of the catheter body, a coil body arranged at the catheter body and the front end tip along the lumen, and a reinforcement body covering the coil body.

US2013/018318 A1 discloses a catheter comprising a catheter body having a lumen, a resin front end tip having a lumen communicating with the lumen of the catheter body and attached to a front end of the catheter body, a coil body arranged at the catheter body tip along the lumen, and a reinforcement body covering the coil body.

### SUMMARY OF THE INVENTION

### TECHNICAL PROBLEM

However, in the case of the conventional catheter, even though a braid is arranged inside the catheter body and the front end tip, a front end tip may be detached from a catheter body during procedures and left inside the patient's body.

The present invention is made in view of the above circumstances. An objective of the present invention is to provide a catheter in which the remaining of a front end tip alone in the patient's body may be prevented even in a case where the front end tip is detached from a catheter body.

### SOLUTION TO PROBLEM

The above objective is achieved by a catheter comprising: a catheter body having a lumen; a resin front end tip having a lumen communicating with the lumen of the catheter body, and attached to a front end of the catheter body; and a coil body and/or a braid arranged at the catheter body and the front end tip along the lumen; wherein
a reinforcement body covering the coil body and/or the braid is arranged only within the front end tip,
the coil body and/or the braid protrudes from the reinforcement body towards a front end of the front end tip,
the reinforcement body is fixed to the front end tip and the coil body and/or the braid,
the reinforcement body is provided as a separate body from the catheter body, and
the reinforcement body comprises a resin harder than that constituting the front end tip.

Further, the above reinforcement body may have a diameter larger than that of the above coil body and/or the above braid.

Further, the above reinforcement may have body has a protrusion part extending toward the outer periphery of the above front end tip.

Furthermore, the front end tip may have a tapered outer periphery in which the diameter is gradually decreased toward a forefront part of the catheter.

### ADVANTAGEOUS EFFECT OF THE INVENTION

According to the present invention, the remaining of the front end tip alone in the patient's body can be prevented even in a case where the front end tip is detached from the catheter body.

Further, when the reinforcement body has a diameter larger than that of the coil body and/or the braid, the remaining of the front end tip alone in the patient's body can further be prevented even in a case where the front end tip is detached from the catheter body.

Further, when the reinforcement body has a protrusion part extending toward the outer periphery of the front end tip, the remaining of the front end tip alone in the patient's body can further be prevented even in a case where the front end tip is detached from the catheter body.

### BRIEF DESCRIPTION OF THE DRAWINGS

Fig. 1 shows a front view illustrating an embodiment of the catheter according to the present invention.
Fig. 2 shows a partial cross sectional view of the catheter according to the first embodiment of the present invention.
Fig. 3 shows a partial cross sectional view of a catheter according to a second embodiment of the present invention.

### DESCRIPTION OF EMBODIMENTS

Below, the present invention will be described with reference to the drawings.

### (First embodiment)

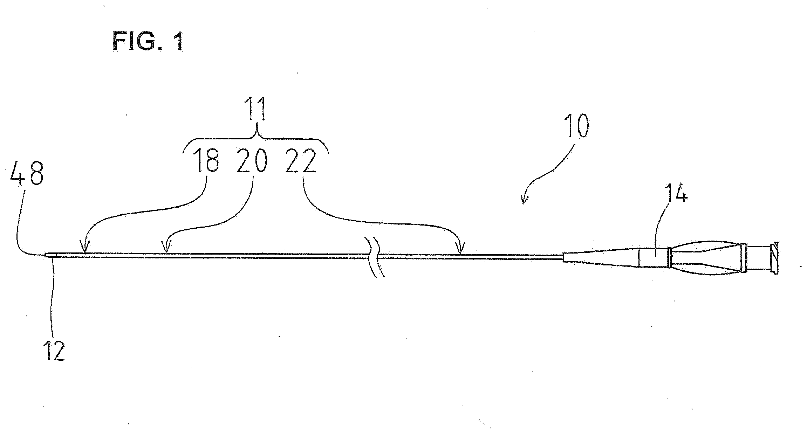

First, a first embodiment of the present invention will be described. Fig. 1 shows a front view illustrating an embodiment of a catheter according to the present invention, and Fig. 2 shows a partial cross-sectional view of the catheter according to the first embodiment of the present invention.

In Fig. 1, a catheter 10 according to this embodiment comprises a catheter body 11, a flexible front end tip 12 attached to the front end of the above catheter body 11 and a connector 14 attached to the base end of the catheter body 11.

More specifically, the catheter body 11 comprises a hollow elongated member having a lumen 16 into which a guide wire will be inserted. In this embodiment, the full length of the catheter body 11 is about 1500 mm. A portion for about 200 mm from the front end of the catheter body 11 corresponds to a front end part 18 having an outer diameter of about 0.88 mm (0.85 to 0.90 mm), and a portion for about 600 mm from the base end of the front end part 18 corresponds to an intermediate part 20 having an outer diameter slightly larger than that of the front end part 18. Further, a portion from the base end of the intermediate part 20 to the connector 14 corresponds to a base end part 22 having an outer diameter even larger than that of intermediate part 20.

Moreover, as shown in Fig. 2, the catheter body 11 has a hollow coil body 24, an inner resin layer 26 covering the inner periphery of the coil body 24 and an outer resin layer 28 covering the outer periphery of the coil body 24.

The inner resin layer 26 has a tube-like shape extending throughout the full length at the innermost part of the catheter body 11. In addition, the lumen 16 of the catheter body 11 is formed in the inner cavity of the inner resin layer 26. Moreover, the inner resin layer 26 axially extends from the front end of the catheter body 11 to the side of the front end.

Note that there is no particular limitation for resin materials for forming the aforementioned inner resin layer 26, but resin materials conferring flexibility and moderate plasticity will appropriately be selected. Among these, for example, polytetrafluoroethylene (PTFE), which has excellent lubricity, is preferably used in view of the slideability of a guide wire.

The coil body 24 is a hollow twisted wire coil in which two or more metal element wires 40 each having a circular cross section are twisted (10 element wires in this embodiment). Further, since the coil body 24 is heat-treated by the known method after the two or more metal element wires 40 are twisted, the residual stress of the coil body 24 is removed.

There is no particular limitation for a metal material for forming the metal element wires 40 of the coil body 24. For example, a metal material such as stainless steel and a superelastic alloy such as a Ni-Ti alloy may each independently be used to form the coil body 24, or they may be used in combination to form the coil body.

The outer resin layer 28, which constitutes the outermost layer of the catheter body 11, extends throughout the full length of the catheter body 11 in a state where the outer periphery of the coil body 24 is covered such that the outer periphery of the metal element wires 40 which constitutes the coil body 24 is not be exposed outside from the outer surface of the catheter body 11.

Further, in the outer resin layer 28, the thickness of a portion corresponding to the outermost layer of the intermediate part 20 of the catheter body 11 is larger than that of a portion corresponding to the outermost layer of the front end part 18 of the catheter body 11. Moreover, the thickness of a portion corresponding to the outermost layer of the base end part 22 of the catheter body 11 is larger than that of a portion corresponding to the outermost layer of the intermediate part 20 of the catheter body 11.

Further, the hardness of the outer resin layer 28 at the front end part 18, the intermediate part 20 and the base end part 22 of the catheter body 11 is increased from the front end part 18 to the base end part 22 in incremental steps. Thereby, the plasticity of the base end part 22, the intermediate part 20 and the front end part 18 of the catheter body 11 is increased from the base end part 22 to the front end part 18 in incremental steps.

There is no particular limitation for a resin material for forming the outer resin layer 28, but a resin material conferring flexibility and moderate plasticity may appropriately be selected. Materials for forming the aforementioned outer resin layer 28 can include polyamide elastomers and the like.

Meanwhile, the front end tip 12 provided at the front end of the catheter body 11 has an elongated tubular overall shape, and comprises a lumen communicating with the lumen 16 of the catheter body 11, and has a plasticity well higher than that of the catheter body 11. In addition, the above front end tip 12 has a tapered outer periphery in which the diameter is gradually decreased toward a forefront part 48.

There is also no particular limitation for a resin material forming the front end tip 12, but a resin material having higher plasticity than that for forming the inner resin layer 26 and the outer resin layer 28 may suitably be used. Examples of such a resin material can include polyurethane elastomers. Note that in this embodiment, tungsten powder is mixed into the front end tip 12. Thereby, the visibility of the front end tip 12 under radioscopy is enhanced advantageously.

Moreover, the inner periphery of the front end tip 12 is formed with the inner resin layer 26 extending from the front end of the catheter body 11, and the coil body 24 which continues from the front end of the catheter body 11 is wound and embedded around the outer periphery of the inner resin layer 26 in the inside of the front end tip 12.

Moreover, in order to prevent the detachment of the front end tip 12 from the coil body 24, a reinforcement body 30 comprising a resin harder than a resin material of the front end tip 12 is fixed to the front end tip 12 and the coil body 24 in the inside of the front end tip 12. Thereby, the reinforcement body 30 fixed to the front end tip 12 and the coil body 24 can prevent the detachment of the front end tip 12 from the coil body 24 even in a case where the front end tip 12 is detached from the catheter body 11, which in turn, can prevent the detachment of the front end tip 12 from the catheter body 11.

Note that, in this embodiment, the reinforcement body 30 is provided as a separate body from the catheter body 11. Further, in this embodiment, the reinforcement body 30 does not have an after-mentioned protrusion part extending toward the outer periphery of the front end tip, but may be formed to have a protrusion part extending toward the outer periphery in order to further prevent the detachment of the front end tip 12 from the catheter body 11 in the axial direction of the catheter.

Examples of a material for the reinforcement body 30 can include polyamide elastomers. In this embodiment, a polyamide elastomer harder than the outer resin layer 28 is used.

### (Second embodiment)

Next, a second embodiment of the present invention will be described. A catheter according to the second embodiment represents an embodiment in which the coil body 24 of the catheter according to the first embodiment is replaced with a braid body 124. Further, since the catheter according to the second embodiment has the same appearance as the catheter 10 according to the first embodiment, only the internal structure is described with reference to a drawing with an overall view omitted.

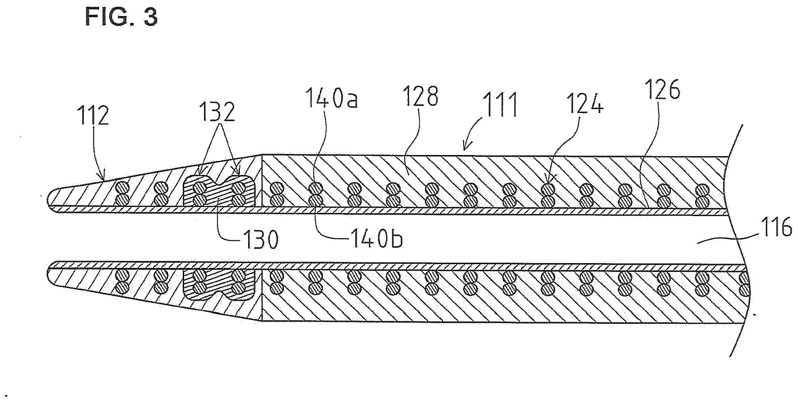

Fig. 3 shows a partial cross sectional view of the catheter according to the second embodiment of the present invention. In Fig. 3, a catheter body 111 comprises a hollow elongated member having a lumen 116 into which a guide wire will be inserted. As shown in Fig. 3, the catheter body 111 has a braid body 124, an inner resin layer 126 covering the inner periphery of the braid body 124 and an outer resin layer 128 covering the outer periphery of the braid body 124.

The inner resin layer 126 has a tube-like shape extending throughout the full length at the innermost part of the catheter body 111. Moreover, the inner resin layer 126 axially protrudes from the front end of the catheter body 111 to the side of the front end. Note that for a resin material for forming the aforementioned inner resin layer 126, a similar material to the inner resin layer 26 in the first embodiment may be used.

The braid body 124 has a structure in which two metal element wires 140a and 140b are woven each other in such a way to form a mesh-like structure. In addition, the braid body 124 having the aforementioned mesh-like structure is wound around the outer periphery of the inner resin layer 126 throughout the full length.

Further, as in the inner resin layer 126, the braid body 124 is wound around the outer periphery of the inner resin layer 126 which axially extends from the front end of the catheter body 111 to the side of the front end, and protruded from the front end of the catheter body 111.

There is no particular limitation for the type of a metal material for the metal element wires 140a and 140b of the braid body 124, but, for example, a wire material comprising tungsten, stainless steel and the like may suitably be used. In addition, a tungsten wire material, which has good visibility under radioscopy, is more suitably used.

Note that there is also no particular limitation for the size of the metal element wires 140a and 140b, but it may appropriately selected depending on the sizes (outer diameters) of the catheter body 111 and the front end tip 112 and the like. According to this embodiment, the diameters of the metal element wires 140a and 140b are about 0.023 mm. In addition, one metal element wire 140a and one metal element wire 140b are woven each other to form the braid body 124.

The outer resin layer 128 constitutes the outermost layer of the catheter body 111. In addition, it extends throughout the full length of the catheter body 111 in a state where the braid body 124 is covered such that the metal element wires 140a and 140b constituting the braid body 124 are not exposed outside from the outer surface of the catheter body 111.

Note that for a resin material for forming the outer resin layer 128, a similar material to the outer resin layer 28 in the first embodiment may be used.

Further, the front end tip 112 provided at the front end of the catheter body 111 has an elongated tubular overall shape, and comprises a lumen communicating with the lumen 116 of the catheter body 111, and has a plasticity well higher than that of the catheter body 111. In addition, the front end tip 112 has a tapered outer periphery in which the diameter is gradually decreased toward a forefront part 48.

Note that for a resin material for forming the front end tip 112, a similar material to the front end tip 12 in the first embodiment may be used.

The inner periphery of the front end tip 112 is formed with the inner resin layer 126 extending from the front end of the catheter body 111, and the braid body 124 which continues from the front end of the catheter body 111 is wound and embedded around the outer periphery of the inner resin layer 126 in the inside of the front end tip 112.

Further, in order to prevent the detachment of the front end tip 112 from the braid body 124, a reinforcement body 130 comprising a resin harder than a resin material of the front end tip 112 is fixed to the front end tip 112 and the coil body 124 in the inside of the front end tip 112. Thereby, the reinforcement body 130 fixed to the front end tip 112 and the braid body 124 can prevent the detachment from the braid body 124 even in a case where the front end tip 112 is detached from the catheter body 111, which, in turn, can prevent the detachment of the front end tip 112 from the catheter body 111.

Then, the reinforcement body 130 may be any as long as it can be fixed to the front end tip 112 and the coil body 124, but it is preferably formed in such a way to have a protrusion part 132 extending toward the outer periphery in order to effectively prevent the detachment of the front end tip 112 from the catheter body 111 in the axial direction of the catheter.

Note that the protrusion part 132 is provided at two positions in the reinforcement body 130 according to this embodiment, but may be provided at one position or at three or more positions.

The embodiments of the present invention are described above in detail, but the present invention shall not be construed in any limitative manner despite of the specific description about the above embodiments.

For example, in the embodiments described above, all the reinforcement bodies are each illustrated to have a larger diameter than the coil body or the braid body, but the reinforcement body does not necessarily have a lager diameter than the coil body or the braid body.

Nonetheless, in a case where the reinforcement body has a larger diameter than the coil body or the braid body, the detachment of the front end tip from the coil body or the braid body can further be prevented.

Moreover, the coil body in the aforementioned embodiments comprises 10 metal element wires having the same diameter. However, the coil body, for example, may comprise 8 metal element wires having the same diameter and the rest 2 metal element wires having a different diameter or may comprise only a single metal element wire.

Nonetheless, two or more element wires are preferably used to form a coil body because the elongation of the coil body can be reduced in case that the front end tip is detached from the catheter body.

Moreover, for the coil body in the aforementioned embodiments, each metal element wire constituting the coil body is arranged in such a way to be spaced in the axial direction of the catheter body, showing a so-called sparsely wound sate. Nonetheless, in a case where the enhanced stiffness of a catheter is preferred, or the enhanced torque transmittability from the base end of a catheter to the front end is preferred, a so-called densely wound sate is preferred in which each metal element wires is arranged in such a way to make contact with each other.

Further, the braid body in the aforementioned embodiments is formed with two inter-woven metal element wires having the same diameter, but it may be formed with a braid body comprising metal element wires having different diameters. Further the braid body may be formed not only with two inter-woven metal element wires but also with more than two inter-woven metal element wires, and may be formed with clockwise metal element wires and counter-clockwise metal element wires, the numbers of the clockwise metal element wires and the counter-clockwise metal element wires being different.

Moreover, the element wires constituting the braid body in the aforementioned embodiments are metal element wires, but the braid body may be formed with woven resin element wires, or may be formed with a metal element wire and a resin element wire woven each other in combination. Furthermore, the number of element wires and the braiding manner of element wires to form a braid body may appropriately be altered.

Further, the metal element wire which constitutes the coil body and the braid body in the aforementioned embodiments has a circular cross section, but the coil body and the braid body may be formed with an element wire with an elliptical cross section or an element wire with a rectangular cross section.

Moreover, the protrusion part in the aforementioned embodiments is described to have a curved shape on the whole, but not limited to it. For example, the protrusion part may have a sharply pointed shape on the front end tip, or may enter into the front end tip in a wedge-like manner.

Furthermore, in the aforementioned embodiments, the coil body or the braid is arranged at the outer periphery of the inner resin layer in view of that the coil body and the braid can easily be formed. Nonetheless, the inner resin layer may be integrally formed with the outer resin layer, and then a coil body or a braid may be arranged in the inside of an additional resin layer. Further, in addition to the two-layered structure of an inner resin layer and an outer resin layer, even a configuration in which a coil body or a braid is arranged in a certain layer of a multilayered structure having three or more layers can be used for the present invention.

Moreover, a configuration in which a coil body alone or a braid alone is arranged in each catheter is described in the aforementioned embodiments, but even a configuration in which a coil body and a braid are both arranged in one catheter can be used for the present invention.

### DESCRIPTION OF SYMBOLS

- 10: Catheter
- 11, 111: Catheter body
- 12, 112: Front end tip
- 14: Connector
- 16, 116: Lumen
- 18: Front end part
- 20: Intermediate part
- 22: Base end part
- 24: Coil body
- 26, 126: Inner resin layer
- 28, 128: Outer resin layer
- 30, 130: Reinforcement body
- 40, 140a, 140b: Metal element wire
- 48: Forefront Part
- 124: Braid body
- 132: Protrusion part

## Claims

1. A catheter (10) comprising:
a catheter body (11, 111) having a lumen (16, 116),
a resin front end tip (12, 112) having a lumen communicating with the lumen (16, 116) of the catheter body (11, 111), and attached to a front end of the catheter body (11, 111), and
a coil body (24) and/or a braid (124) arranged at the catheter body (11, 111) and the front end tip (12, 112) along the lumen (16, 116),
**characterized in that**
a reinforcement body (30, 130) covering the coil body (24) and/or the braid (124) is arranged only within the front end tip (12, 112),
the coil body (24) and/or the braid (124) protrudes from the reinforcement body (30, 130) towards a front end of the front end tip (12, 112),
the reinforcement body (30, 130) is fixed to the front end tip (12, 112) and the coil body (24) and/or the braid (124),
the reinforcement body (30, 130) is provided as a separate body from the catheter body (11, 111), and
the reinforcement body (30, 130) comprises a resin harder than that constituting the front end tip (12, 112).

2. The catheter (10) according to claim 1, **characterized by** that the reinforcement body (30, 130) has a diameter larger than that of the coil body (24) and/or the braid (124).

3. The catheter (10) according to claim 2, **characterized by** that the reinforcement body (130) has a protrusion part (132) extending toward an outer periphery of the front end tip (112).

## Patentansprüche

1. Katheter (10) mit:
einem Katheterkörper (11, 111), der ein Lumen (16, 116) aufweist,
einer vorderen Spitze (12, 112) aus Harz, die ein Lumen aufweist, das mit dem Lumen (16, 116) des Katheterkörpers (11, 111) kommuniziert, und die an einem vorderen Ende des Katheterkörpers (11, 111) angebracht ist, und
einem Wicklungskörper (24) und/oder Geflecht (124), der oder das am Katheterkörper (11, 111) und an der vorderen Spitze (12, 112) entlang des Lumens (16, 116) angeordnet ist, **dadurch gekennzeichnet, dass**
ein Versteifungskörper (30, 130), der den Wicklungskörper (24) und/oder das Geflecht (124) umhüllt, nur in der vorderen Spitze (12, 112) angeordnet ist,
der Wicklungskörper (24) und/oder das Geflecht (124) aus dem Versteifungskörper (30, 130) heraus in Richtung eines vorderen Endes der vorderen Spitze (12, 112) ragt,
der Versteifungskörper (30, 130) an der vorderen Spitze (12, 112) und am Wicklungskörper (24) und/oder Geflecht (124) befestigt ist,
der Versteifungskörper (30, 130) als ein vom Katherkörper (11, 111) separater Körper vorgesehen ist, und
der Versteifungskörper (30, 130) ein Harz aufweist, das härter ist als das Harz, aus dem die vordere Spitze (12, 112) gebildet ist.

2. Katheter (10) nach Anspruch 1, **dadurch gekennzeichnet, dass** der Versteifungskörper (30, 130) einen Durchmesser hat, der größer ist als der Durchmesser des Wicklungskörpers (24) und/oder Geflechts (124).

3. Katheter (10) nach Anspruch 2, **dadurch gekennzeichnet, dass** der Versteifungskörper (130) einen sich in Richtung eines Außenumfangs der vorderen Spitze (112) erstreckenden Vorsprungsteil (132) hat.

## Revendications

1. Cathéter (10) comprenant :
un corps de cathéter (11, 111) ayant une lumière (16, 116),
une pointe d'extrémité avant en résine (12, 112) ayant une lumière communiquant avec la lumière (16, 116) du corps de cathéter (11, 111), et fixée sur une extrémité avant du corps de cathéter (11, 111), et
un corps de bobine (24) et/ou une tresse (124) agencé(s) au niveau du corps de cathéter (11, 111) et la pointe d'extrémité avant (12, 112) le long de la lumière (16, 116),
**caractérisé en ce que**
un corps de renforcement (30, 130) recouvrant le corps de bobine (24) et/ou la tresse (124) est agencé uniquement à l'intérieur de la pointe d'extrémité avant (12, 112),
le corps de bobine (24) et/ou la tresse (124) fait saillie à partir du corps de renforcement (30, 130) vers une extrémité avant de la pointe d'extrémité avant (12, 112),
le corps de renforcement (30, 130) est fixé à la pointe d'extrémité avant (12, 112) et au corps de bobine (24) et/ou la tresse (124),
le corps de renforcement (30, 130) est fourni comme un corps séparé du corps de cathéter (11, 111), et
le corps de renforcement (30, 130) comprend une résine plus dure que celle constituant la pointe d'extrémité avant (12, 112).

2. Cathéter (10) selon la revendication 1, **caractérisé en ce que** le corps de renforcement (30, 130) présente un diamètre plus large que celui du corps de bobine (24) et/ou la tresse (124).

3. Cathéter (10) selon la revendication 2, **caractérisé en ce que** le corps de renforcement (130) a une partie de saillie (132) s'étendant vers une périphérie externe de la pointe d'extrémité avant (112).
